(19) **Europäisches Patentamt / European Patent Office / Office européen des brevets**

(11) **EP 4 548 943 A1**

(12) **EUROPEAN PATENT APPLICATION**
published in accordance with Art. 153(4) EPC

(43) Date of publication:
**07.05.2025 Bulletin 2025/19**

(21) Application number: **23831505.5**

(22) Date of filing: **28.06.2023**

(51) International Patent Classification (IPC):
*A61M 1/14* (2006.01)        *A61M 1/16* (2006.01)

(52) Cooperative Patent Classification (CPC):
**A61M 1/14; A61M 1/16**

(86) International application number:
**PCT/JP2023/023961**

(87) International publication number:
**WO 2024/005065 (04.01.2024 Gazette 2024/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.06.2022  JP 2022103785**

(71) Applicant: **Nikkiso Co., Ltd.**
**Tokyo 150-6022 (JP)**

(72) Inventor: **KATAYAMA, Yuki**
**Makinohara-shi Shizuoka 421-0496 (JP)**

(74) Representative: **Grosse Schumacher Knauer von Hirschhausen**
**Patent- und Rechtsanwälte**
**Nymphenburger Strasse 14**
**80335 München (DE)**

(54) **BLOOD PURIFICATION DEVICE**

(57)    A blood purification device, including a blood circuit to extracorporeally circulate blood of a patient through a blood purifier, a tube unit through which a liquid including a dialysate as a liquid type flows and into which a predetermined component removed from the blood circulating extracorporeally in the blood circuit is discharged, a display unit that displays a liquid image imitating a liquid as a background image, and a control unit that controls processing of a fluid flowing through at least one of the blood circuit and the tube unit, and also controls the display unit to change a height of a liquid surface of the liquid image in the background image according to a processing status of the fluid.

**FIG. 1A**

**Description**

TECHNICAL FIELD

The present invention relates to a blood purification device.

BACKGROUND OF THE INVENTION

As a conventional technique, a display system for blood purification device is known, which includes a screen to display the contents of each process of the blood purification device and is configured so that the background color of the screen can be set to a different background color for each process (see, e.g., Patent Literature 1).

Since this conventional display system for blood purification device changes the background color for each process, it is possible to clearly grasp the current process at a glance.

CITATION LIST

PATENT LITERATURES

Patent Literature 1: JP 2004-313324A

SUMMARY OF THE INVENTION

This conventional display system for blood purification device changes only the background color for each process, and the processing status of the current process cannot be visually checked from only the background.

Therefore, it is an object of the invention to provide a blood purification device with which the processing status of blood purification treatment is visually checked easily.

To achieve the object above, the invention provides:

a blood circuit to extracorporeally circulate blood of a patient through a blood purifier;

a tube unit through which a liquid comprising a dialysate as a liquid type flows and into which a predetermined component removed from the blood circulating extracorporeally in the blood circuit is discharged;

a display unit that displays a liquid image imitating a liquid as a background image; and

a control unit that controls processing of a fluid flowing through at least one of the blood circuit and the tube unit, and also controls the display unit to change a height of a liquid surface of the liquid image in the background image according to a processing status of the fluid.

Advantageous Effects of the Invention

According to the invention, the processing status of blood purification treatment can be easily visually checked.

BRIEF DESCRIPTION OF DRAWINGS

Fig. 1A shows an example of a display/input unit of a blood purification device.

Fig. 1B is an explanatory diagram illustrating an example of a background image.

Fig. 2A is an example block diagram illustrating the blood purification device.

Fig. 2B is an explanatory diagram illustrating an example of dialysate and blood flows.

Fig. 3A is an explanatory diagram illustrating an example of progress level display of the background image.

Fig. 3B is a diagram illustrating an example of the background image when blood purification treatment is stopped.

Fig. 4A is a diagram illustrating an example of transition of the background image from the beginning to the end of water removal processing.

Fig. 4B is a diagram illustrating an example of transition of the background image from the beginning to the end of the water removal processing.

Fig. 4C is a diagram illustrating an example of transition of the background image from the beginning to the end of the water removal processing.

Fig. 4D is a diagram illustrating an example of transition of the background image from the beginning to the end of the water removal processing.

Fig. 5A is a diagram illustrating an example of changes in a liquid image at different water removal rates.

Fig. 5B is a diagram illustrating an example of changes in a liquid image at different water removal rates.

Fig. 5C is a diagram illustrating an example of changes in a liquid image at different water removal rates.

Fig. 5D is a diagram illustrating an example of changes in a liquid image at different water removal rates.

Fig. 5E is a diagram illustrating an example of changes in a liquid image at different water removal rates.

Fig. 5F is a diagram illustrating an example of changes in a liquid image at different water removal rates.

Fig. 5G is a diagram illustrating an example of changes in a liquid image at different water removal rates.

Fig. 6A is an explanatory diagram illustrating an example of a change in the liquid image in a modification.

Fig. 6B is an explanatory diagram illustrating an example of the change in the liquid image in the modification.

Fig. 6C is an explanatory diagram illustrating an example of the change in the liquid image in the modification.

Fig. 7A is a diagram illustrating an example of when a toxin image is superimposed on the liquid image in a modification.

Fig. 7B is a diagram illustrating an example of when the toxin image is superimposed on the liquid image in the modification.

Fig. 8 is a flowchart showing an example of the water removal processing of the blood purification device.

Fig. 9A is an example of a perspective view showing the blood purification device when an extracorporeal circulation unit is attached to a dialysate supply/discharge unit.

Fig. 9B is a perspective view when the extracorporeal circulation unit is detached from the dialysate supply/discharge unit.

Fig. 10 is an example block diagram illustrating the blood purification device in the case where the extracorporeal circulation unit and the dialysate supply/discharge unit are separate units.

## DETAILED DESCRIPTION OF THE INVENTION

Embodiment

General configuration of Blood purifying device 1

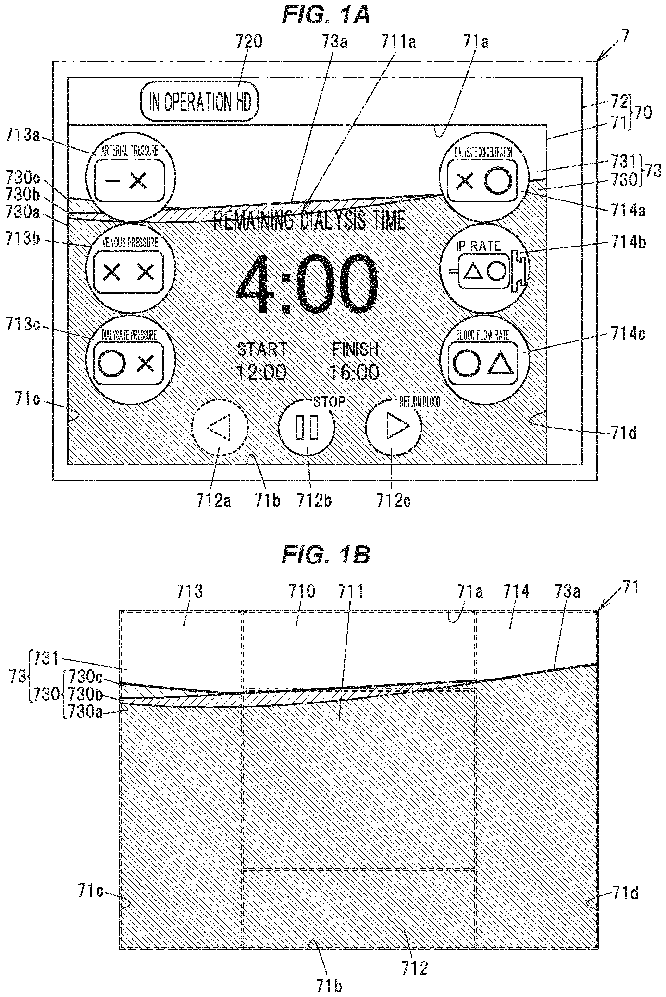

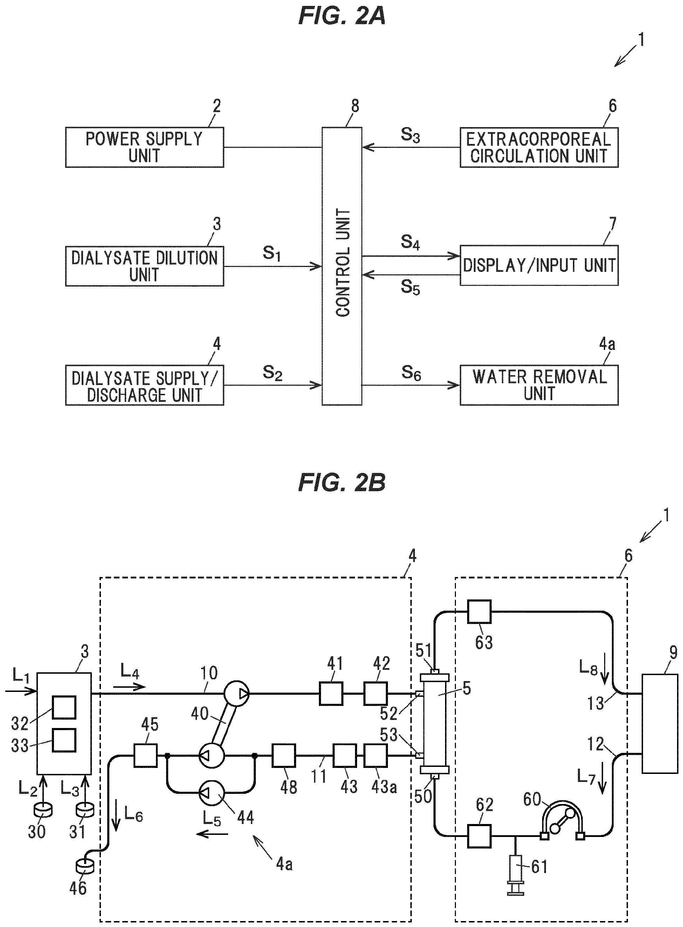

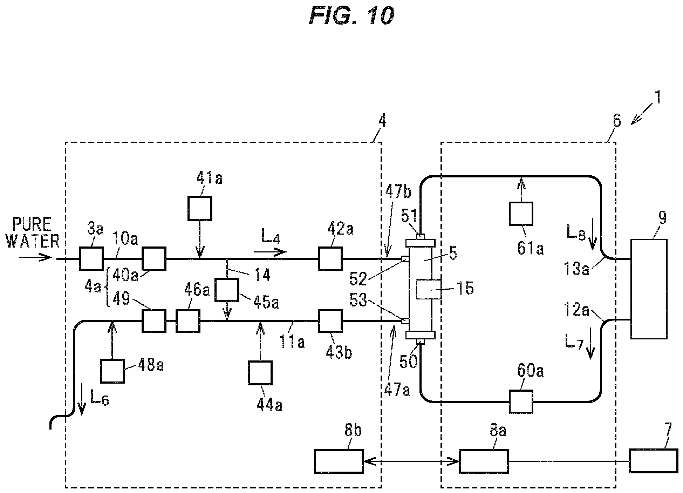

Fig. 1A shows an example of a display/input unit of a blood purification device in an embodiment, and Fig. 1B is an explanatory diagram illustrating an example of a background image displayed on the display/input unit. Fig. 2A is an example block diagram illustrating the blood purification device in the embodiment, and Fig. 2B is an explanatory diagram illustrating an example of dialysate and blood flows in the blood purification device. In the drawings of the embodiment described below, a scale ratio or shape may be different between the drawings or different from an actual ratio or shape. In addition, in Fig. 2A, flows of main information are indicated by arrows. Furthermore, in Figs. 2B and 10, flows of main fluids are indicated by arrows.

A blood purification device 1 in the present embodiment is configured to change a background image 73, which is displayed on a display/input unit 7 as a display unit, according to a processing status of blood purification treatment so that patients and medical staff can easily visually check the processing statuses of preparation for blood purification treatment, during blood purification treatment, and of post-processing of blood purification treatment even from a distance, as shown in Figs. 1A and 1B.

In particular, the blood purification device 1 generally includes a blood circuit to extracorporeally circulate blood $L_7$ of a patient 9 through a dialyzer 5 as a blood purifier, a dialysate supply/discharge unit 4 as a tube unit through which a liquid including a dialysate $L_4$ as a liquid type flows and into which a predetermined component removed from the blood $L_7$ circulating extracorporeally in the blood circuit is discharged, the display/input unit 7 that displays a liquid image 730 imitating a liquid as the background image 73, and a control unit 8 that controls processing of a fluid flowing through at least one of the blood circuit and the dialysate supply/discharge unit 4, and also controls the display/input unit 7 to change a height of a liquid surface 73a of the liquid image 730 in the background image 73 according to a fluid processing status in blood purification treatment. An arterial blood circuit 12 and a venous blood circuit 13 (which are described later) constitute the blood circuit. The term "liquid including a dialysate $L_4$ as a liquid type" means that the liquid includes the dialysate $L_4$ as a type of liquid, not as a liquid component.

The control unit 8 is configured to control the display/input unit 7 to change the height of the liquid surface 73a of the liquid image 730 and also to continuously ripple the liquid surface 73a, according to the fluid processing status.

Furthermore, the control unit 8 is configured to control execution of blood purification treatment for exchange of components between the blood $L_7$ flowing through the blood circuit and the dialysate $L_4$ flowing through the dialysate supply/discharge unit 4 and, when the blood purification treatment is cancelled or suspended, controls the display/input unit 7 to stop fluctuation of the liquid surface 73a of the liquid image 730.

Still further, the control unit 8 is configured to cause the display/input unit 7 to display a display target image, which is to be displayed during the blood purification treatment, so as to be superimposed on the liquid image 730.

The liquid image 730 is an image imitating water and is colored light blue as an example, but is not limited thereto and may be an image that imitates another liquid with a different viscosity or color, etc. The height of the liquid surface 73a, i.e., the water level, is a height from a bottom surface 71b of a main display area 71 (described later), as shown in Fig. 1A. Furthermore, as an example, display items 713a to 713c and display items 714a to 714c (described later) are the display target images, as shown in Fig. 1A.

The blood purification device 1 further includes a water removal unit 4a that, when driven, removes water from the blood $L_7$ extracorporeally circulating in the blood circuit so that the water is discharged into the dialysate supply/discharge unit 4. The fluid processing status is a status of water removal processing by the water removal unit 4a. The control unit 8 changes the liquid image 730 according to the status of the water removal processing by the water removal unit 4a.

The fluid processing includes water removal or removal of waste matters during blood purification treatment, gas purging in preparation for treatment, and cleaning/disinfection of the dialysate supply/discharge unit 4 after treatment. The fluid processing status includes a water removal processing status during blood purification treatment, a processing status of removal of waste matters (urea, etc.) as predetermined components or of blood cell/plasma components, a processing status of gas purging, and a processing status of cleaning. Thus, the fluid is the dialysate $L_4$ or the blood $L_7$, etc. that flows through at least one of the blood circuit and dialysate supply/discharge unit 4 in these processings.

As shown in Fig. 2A, the blood purification device 1 further includes a power supply unit 2, a dialysate dilution unit 3, and an extracorporeal circulation unit 6.

Configuration of Power supply unit 2

The power supply unit 2 is configured to supply power necessary for the blood purification device 1 to operate.

Configuration of Dialysate dilution unit 3

As shown in Fig. 2B, the dialysate dilution unit 3 is generally configured to create the dialysate $L_4$ by using RO (Reverse Osmosis) water $L_1$ supplied from the outside, an undiluted A-solution $L_2$ supplied from an undiluted A-solution tank 30 and an undiluted B-solution $L_3$ supplied from an undiluted B-solution 31, and adjusting a concentration of a chemical liquid.

The dialysate dilution unit 3 includes a concentration meter 32 to measure a concentration of the produced dialysate $L_4$, and a thermometer 33 to measure a temperature of the dialysate $L_4$. The concentration meter 32 is constructed using, e.g., a conductivity cell, etc. The thermometer 33 is constructed using, e.g., a thermistor or a platinum resistance thermometer, etc.

As shown in Fig. 2A, the dialysate dilution unit 3 generates first management information $S_1$ including information of measured concentration and temperature, and outputs it to the control unit 8. The first management information $S_1$ is output at a predetermined cycle as an example, but it is not limited thereto.

Configuration of Dialysate supply/discharge unit 4

As shown in Fig. 2B, the dialysate supply/discharge unit 4 includes a dual pump 40, a pressure gauge 41, a flowmeter 42, a pressure gauge 43, a water removal pump 44, a flowmeter 45, and a pressure pump 48. The water removal unit 4a in the present embodiment is composed of the water removal pump 44, as an example.

As shown in Fig. 2B, the dual pump 40 is arranged over a dialysate introduction line 10 and a dialysate discharge line 11. The dual pump 40 is configured to apply pressure to the dialysate introduction line 10 to send the dialysate $L_4$ to the dialyzer 5 and to apply pressure to the dialysate discharge line 11 to send a dialysate effluent $L_6$ to an effluent tank 46. The dual pump 40 sends the dialysate $L_4$ and the dialysate effluent $L_6$ at the same pressure in the dialysate introduction line 10 and the dialysate discharge line 11.

The pressure gauge 41 and the pressure gauge 43 are, e.g., semiconductor pressure sensors using diaphragms. The pressure gauge 41 measures pressure of the dialysate $L_4$. The pressure gauge 41 is arranged on the dialysate introduction line 10 on the dialyzer 5 side relative to the dual pump 40.

The pressure gauge 43 measures pressure of the dialysate effluent $L_6$. The pressure gauge 43 is arranged on the dialyzer 5 side relative to the dual pump 40.

The flowmeter 42 and the flowmeter 45 are, e.g., flow sensors using ultrasonic waves or laser light. The flowmeter 42 measures a flow rate of the dialysate $L_4$. The flowmeter 42 is arranged on the dialysate introduction line 10 between the pressure gauge 41 and the dialyzer 5. In this regard, the positions of the pressure gauge 41 and the flowmeter 42 may be swapped.

The flowmeter 45 measures a flow rate of the dialysate effluent $L_6$. The flowmeter 45 is arranged after the water removal pump 44 on the dialysate discharge line 11.

The water removal pump 44 is connected so as to bypass the dual pump 40. The water removal pump 44 is provided to remove water $L_5$ from the blood $L_7$ of the patient 9 flowing through the dialyzer 5.

The pressure pump 48 is a cascade pump (a non-positive displacement pump) that feeds the dialysate $L_4$ in the dialysate discharge line 11 to assist the dual pump 40 in liquid feeding. The pressure pump 48 is arranged between the pressure gauge 43 and the water removal pump 44.

The dialysate supply/discharge unit 4 generates second management information $S_2$ including information of the pressures of the dialysate $L_4$ and the dialysate effluent $L_6$ measured by the pressure gauge 41 and the pressure gauge 43 and the flow rates of the dialysate $L_4$ and the dialysate effluent $L_6$ measured by the flowmeter 42 and the flowmeter 45, and outputs the second management information $S_2$ to the control unit 8. The second management information $S_2$ is output at a predetermined cycle as an example, but it is not limited thereto.

Configuration of Dialyzer 5

The dialyzer 5 includes a blood introduction port 50, a blood discharge port 51, a dialysate introduction port 52, and a dialysate discharge port 53. As shown in Fig. 2B, the dialyzer 5 is arranged such that the dialysate introduction port 52 is connected to the dialysate introduction line 10 and the dialysate discharge port 53 is connected to the dialysate discharge line 11. The dialyzer 5 is arranged also such that the blood introduction port 50 is connected to the arterial blood circuit 12 and the blood discharge port 51 is connected to the venous blood circuit 13.

The dialyzer 5 has plural hollow fibers and is configured to purify the blood $L_7$ by the hollow fibers. A blood flow route connecting the blood introduction port 50 and the blood discharge port 51 through a blood purification membrane and a dialysate flow route connecting the dialysate introduction port 52 and the dialysate discharge 53 are also formed in the dialyzer 5. The hollow fibers constituting the blood purification membrane form a hollow fiber membrane with microscopic holes penetrating from an outer peripheral surface to an inner peripheral surface, and impurities, etc., in the blood are transferred into the dialysate $L_4$ through the hollow fiber membrane.

During this purification, excess water in the blood $L_7$ moves to the dialysate $L_4$ side by reducing the pressure on the dialysate introduction line 10 and the dialysate discharge line 11 side to lower than the pressure of the arterial blood circuit 12 and the venous blood circuit 13 using the water removal pump 44 of the water removal unit 4a. Therefore, since the amount of removed water is an amount of the water $L_5$ drawn by the water removal pump 44, the amount of removed water can be measured by operating the water removal pump 44 so that the pressures in the dialysate introduction line 10 and the dialysate discharge line 11 are equal. The water removal rate is proportional to the operating speed of the water removal pump 44 which is controlled based on a control signal $S_6$ output from the control unit 8, hence, the operation may be made faster to increase the water removal rate and may be made slower to decrease the water removal rate.

Configuration of Extracorporeal circulation unit 6

As shown in Fig. 2B, the extracorporeal circulation unit 6 includes a blood pump 60, a syringe pump 61, a flowmeter 62, and a flowmeter 63.

The blood pump 60 is, e.g., a peristaltic pump. The blood pump 60 is configured to send the blood $L_7$ in the arterial blood circuit 12 to the dialyzer 5.

The syringe pump 61 is, e.g., a syringe-shaped pump. The syringe pump 61 is arranged between the blood pump 60 and the flowmeter 62. The syringe pump 61 is configured to introduce an anticoagulant, which suppresses coagulation of the blood $L_7$, into the arterial blood circuit 12.

The flowmeter 62 and the flowmeter 63 are, e.g., flow sensors using ultrasonic waves or laser light. The flowmeter 62 measures a flow rate of the blood $L_7$ flowing through the arterial blood circuit 12. The flowmeter 62 is arranged between the syringe pump 61 and the dialyzer 5. The flowmeter 63 is arranged on the venous blood circuit 13 and measures a flow rate of purified blood $L_8$ flowing through the venous blood circuit 13.

The extracorporeal circulation unit 6 generates third management information $S_3$ including information of the flow rates of the blood $L_7$ and the purified blood $L_8$ measured by the flowmeter 62 and the flowmeter 63 and information of an **IP** rate of the syringe pump 61 which is an introducing rate of the anticoagulant, and outputs the third management information $S_3$ to the control unit 8. The third management information $S_3$ is output at a predetermined cycle as an example, but it is not limited thereto.

Configuration of Display/input unit 7

The display/input unit 7 is configured as, e.g., a touch panel that displays images and also receives touch inputs. The display/input unit 7 displays images on a display screen 70 based on display control information $S_4$ output from the control unit 8. The display/input unit 7 also generates operation information $S_5$ based on the performed operation and outputs it to the control unit 8.

As an example, the display screen 70 has the main display area 71 and a sub-display area 72, as shown in Fig.

1A. As an example, the main display area 71 has, but is not limited to, an upper area 710, a central area 711, a lower area 712, a left area 713, and a right area 714, as shown in Fig. 1B.

The upper area 710 does not display other images to ensure the visibility of the background image 73 in the present embodiment, but may be configured to display other images.

The central area 711 displays a central image 711a as a text image about remaining dialysis time, as an example. The central image 711a with "Remaining dialysis time", "4:00", "Start", "Finish", "12:00" and "16:00" is displayed in the central area 711 in the present embodiment, but it is not limited thereto. When the treatment is finished, the central image 711a with "Finished" or "Ended" is displayed in the central area 711, as an example.

An operation image 712a, an operation image 712b, and an operation image 712c are displayed in the lower area 712 as an example, but it is not limited thereto. The operation images 712a to 712c are displayed including text images related to start and stop, etc., of the blood purification treatment. The text images displayed in these operation images 712a to 712c are "In Operation", "Stopped", "Bypass", "Ready", "Gas Purging", "Return Blood", "Draining", "Cleaning", "Water Cleaning", "Disinfecting" and "Acid Cleaning", etc., as an example.

"In Operation" indicates that the device is in an operating state in a selected treatment mode. "Stopped" indicates that the blood purification device 1 is stopped in the selected treatment mode and can transition to another process or another treatment mode. "Bypass" indicates that the supply of the dialysate $L_4$ to the dialyzer 5 is being cut off. "Ready" indicates that that the blood purification device 1 is in a prepared state in the selected treatment mode. "Gas Purging" indicates that a function to remove air from the dialysate introduction line 10 and the dialysate discharge line 11 is in operation. "Return Blood" indicates that blood return processing can be performed in all treatment modes. "Draining" indicates that a function to discharge the dialysate $L_4$, etc. filling the dialyzer 5 is in operation. "Cleaning" indicates that the blood purification apparatus 1 is in a state of being cleaned with water, disinfected, cleaned with acid, etc., or is transitioning to this process. "Water Cleaning" indicates that cleaning/replacement with clean water is being performed on the dialysate introduction line 10 and the dialysate discharge line 11. "Disinfecting" indicates that cleaning/replacement with hypochlorous acid or a chemical liquid used for similar purposes is being performed on the dialysate introduction line 10 and the dialysate discharge line 11. "Acid Cleaning" indicates that cleaning/replacement with acetic acid, which is used to prevent precipitation and deposition of salts, or a chemical liquid used for similar purposes is being performed on the dialysate introduction line 10 and the dialysate discharge line 11.

The display item 713a, the display item 713b and the display item 713c are displayed in the left area 713, as an example. Then, the display item 714a, the display item 714b and the display item 714c are displayed in the right area 714, as an example. The display items 713a to 713c and the display items 714a to 714c are items that are preferably displayed on the display screen 70 during the blood purification treatment, and are images with numerical values therein. In Fig. 1A, the numerical values are represented by shapes such as "∘" and "×".

The items which are preferably displayed during the blood purification treatment include blood flow rate, dialysate temperature, target water removal amount, current water removal amount, water removal rate, dialysate pressure or TMP (Trans Membrane Pressure), arterial pressure, venous pressure, syringe pump infusion rate, dialysate concentration, and treatment mode, as an example.

In the present embodiment, as shown in Fig. 1A, arterial pressure, venous pressure, dialysate pressure, dialysate concentration, IP rate (syringe pump infusion rate) and blood flow rate are displayed as the display items 713a to 713c and the display items 714a to 714c, as an example. The display of the treatment mode, water removal rate, current water removal amount and target water removal amount will be described later.

For the display items 713a to 713c and the display items 714a to 714c, it is possible to select the number of display items to be displayed and it is possible to set the display positions of the display items.

The sub-display area 72 is a display area on a side of a top surface 71a and a right surface 71d of the main display area 71. On the top surface 71a side, the sub-display area 72 displays a mode image 720 indicating a treatment mode as shown in Fig. 1A. The treatment modes include HD mode, HF mode, HDF mode, and ECUM mode, as an example.

The HD mode is a mode in which the blood purification treatment is performed using the hemodialysis method. The mode image 720 shown in Fig. 1A shows that the HD mode is selected and is in operation. The HF mode is a mode in which the blood purification treatment is performed using the hemofiltration method. The HDF mode is a mode in which the blood purification treatment is performed using the hemodiafiltration method. The ECUM mode is a mode in which the blood purification treatment is performed using the extracorporeal ultrafiltration method.

Configuration of Control unit 8

The control unit 8 is a microcomputer composed of a CPU (Central Processing Unit) performing calculation and processing, etc., of the acquired data according to a stored program, and a RAM (Random Access Memory) and a ROM (Read Only Memory) as semiconductor memories, etc. The control unit 8 is configured to comprehensively control the dialysate dilution unit 3, etc.

The control unit 8 changes the background image 73 and also updates the values displayed in the display items 713a to 713c and the display items 714a to 714c, based on the first management information $S_1$ obtained from the dialysate dilution unit 3, the second management information $S_2$ obtained from the dialysate supply/discharge unit 4, the third management information $S_3$ obtained from the extracorporeal circulation unit 6, and the processing status of the water removal processing by the water removal unit 4a.

In the present embodiment, the background image 73 changes according to the processing status of the water removal processing, i.e., the degree of water removal. The display items for this water removal processing are the target water removal amount, the current water removal amount and the water removal rate, which are preferably displayed on the display/input unit 7. The background image 73 expresses the target water removal amount, the current water removal amount and the water removal rate by the water level of the liquid image 730 or the movement of the liquid surface 73a, not by the numerical values. The control unit 8 changes the liquid surface 73a of the liquid image 730 according to the status of the water removal processing by the water removal unit 4a, i.e., changes in tandem with the speed of the water removal pump 44 of the water removal unit 4a. In more particular, the control unit 8 changes the extent of fluctuation of the liquid surface 73a of the liquid image 730 in tandem with the speed of the water removal pump 44. Next, the background image 73 will be described.

Background image 73

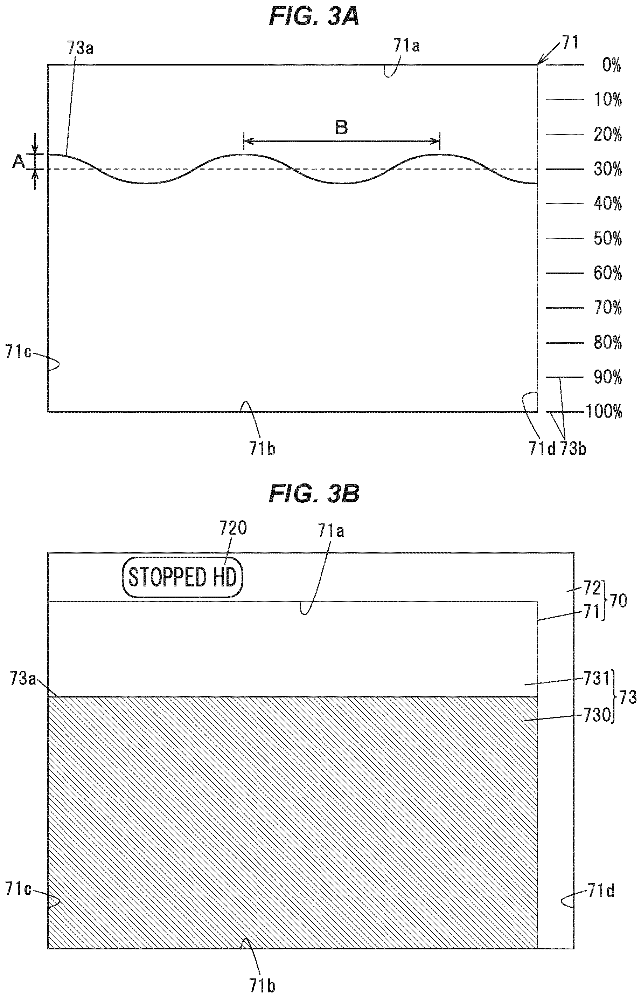

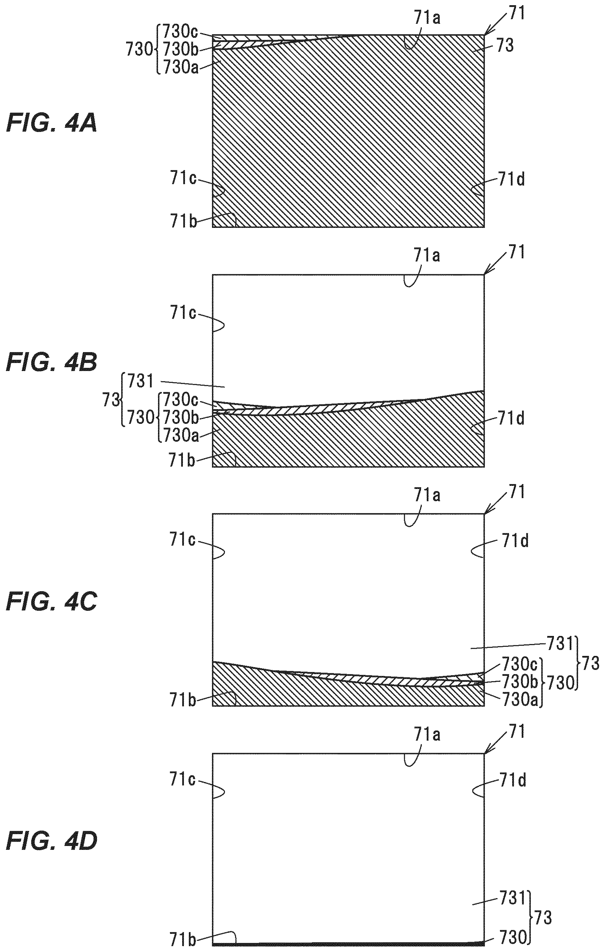

Fig. 3A is an explanatory diagram illustrating an example of progress level display of the background image in the embodiment, and Fig. 3B is a diagram illustrating an example of the background image when the water removal processing is stopped. Figs. 4A to 4D are diagrams illustrating an example of transition of the background image from the beginning to the end of the blood purification treatment in the embodiment.

As shown in Fig. 1B, the background image 73 has the liquid image 730 and an empty space image 731. The liquid image 730 has a first wave image 730a, a second wave image 730b and a third wave image 730c as an example, but is not limited thereto and may have more or less waves as long as waves can be expressed. The empty space image 731 is an image excluding the liquid image 730 in the background image 73. A boundary between the liquid image 730 and the empty space image 731 is the liquid surface 73a. The liquid surface 73a is the outline of the waves and is reminiscent of the surface of water.

As shown in Fig. 1B, the liquid image 730 is configured such that the first wave image 730a is located at the forefront, followed by the second wave image 730b and the third wave image 730c in this order on the deep side of the paper surface, to make the waves appear three-dimensional. In addition, the liquid image 730 is lightened in color in the order of the first wave image 730a, the second wave image 730b, and the third wave image 730c. In the liquid image 730 shown in Fig. 1A, etc., the color strength is expressed by how the spacing between the hatched lines is narrow, where the narrower the spacing, the darker the color.

The control unit 8 is configured to stop the fluctuation of the liquid surface 73a of the liquid image 730 when the blood purification treatment is cancelled or suspended, as shown in Fig. 3B. This liquid surface 73a shows the progress of the blood purification treatment when stopped. Then, an image of "HD Stopped" is displayed in the mode image 720.

The color of the empty space image 731 is white as an example, but is not limited thereto as long as the difference from the liquid image 730 can be visually recognized.

When the fluid processing has display items of target amount, current amount and processing rate, the target amount is represented by an area of the main display area 71. Then, the current amount is represented by an area of the empty space image 731 relative to the main display area 71. Furthermore, the processing rate is represented by a speed of fluctuation of the the liquid image 730.

When the fluid processing is the water removal processing, the water removal processing has display items of the target water removal amount, the current water removal amount and the water removal rate, as described above. The target water removal amount is a value calculated by subtracting a set dry weight from a pre-dialysis body weight of a patient. The current water removal amount is a cumulative amount of water removed since the start of the treatment to the present time. The water removal rate indicates a set value of the water removal rate per hour.

The target water removal amount as the target amount is represented by the area of the main display area 71. The current water removal amount as the current amount is represented by the area of the empty space image 731 relative to the main display area 71. In other words, the liquid image 730 shows a remaining amount of water to be removed. The water removal rate as the processing rate is represented by the speed of the fluctuation of the liquid image 730.

As an example, the control unit 8 is configured to divide the target water removal amount entered through the display/input unit 7 into ten levels between 0 and 100%, as shown in Fig. 3A. The target water removal amount is a value entered in advance through the display/input unit 7. The height of the liquid surface 73a (the water level) showing the current water removal amount is calculated using the following equation and is then rounded off to one of ten levels.

Water level = 100% - ((target water removal amount - remaining amount of water to be removed) / target water removal amount $\times$ 100)

As shown in Figs. 4A and 4B, the control unit 8 positions the liquid surface 73a at the highest water level when the fluid processing status, i.e., the processing status of the water removal processing is 0%, and positions the liquid surface 73a at the lowest water level when the processing status of the water removal processing is 100%.

The height of the liquid surface 73a changes according to the current water removal amount, as shown in Figs. 4A to 4D. The liquid surface 73a, when the current water removal amount is 10 to 90% of the target water removal amount, rises and falls in waves, centered at a scale mark 73b of 10 to 90% shown in Fig. 3A, as an example. For example, Fig. 3A shows the liquid surface 73a oscillating around the scale mark 73b of 30%.

As an example, the liquid surface 73a, when the current water removal amount is 0%, rises and falls in waves, with the scale mark 73b of 0% as the upper limit, as shown in Figs. 3A and 4A.

Then, as an example, the liquid surface 73a, when the current water removal amount is 100%, rises and falls in waves, with the scale mark 73b of 100% as the lower limit, as shown in Figs. 3A and 4D.

The control unit 8 also changes the fluctuation speed of the liquid surface 73a of the liquid image 730 according to the water removal rate. For example, when the water removal rate is fast, the control unit 8 causes the liquid image 730 to change faster than when the rate is slow. The water removal rate and the water removal amount are values that are determined when either one is set.

$$\text{Water removal rate (L/h)} = \text{Water removal amount (L)} / \text{Treatment time (h)}$$

$$\text{Water removal amount (mL)} / \text{Water removal rate (L/h)} \times \text{Treatment time (h)}$$

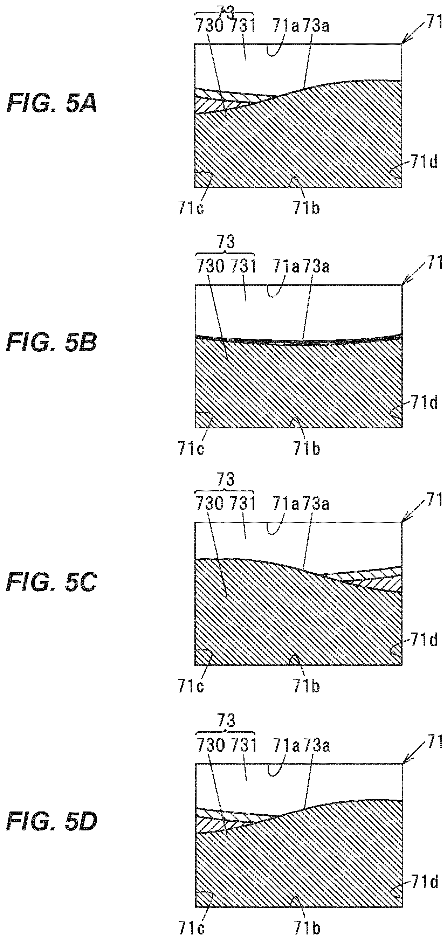

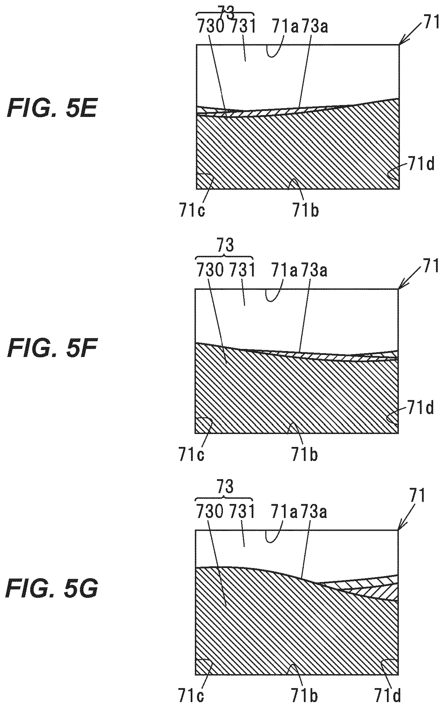

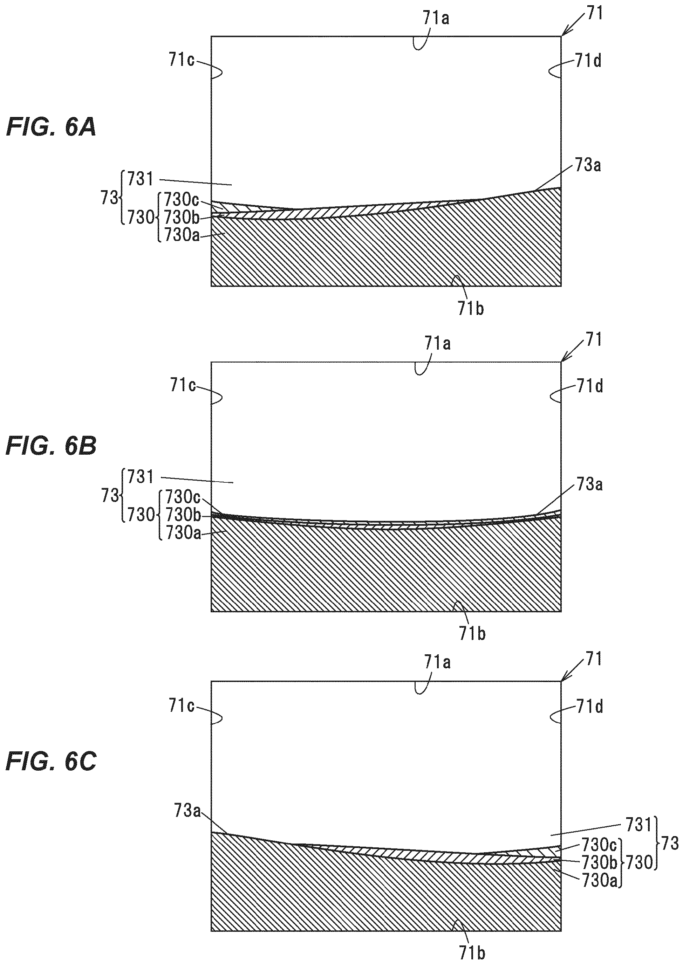

Figs. 5A to 5C and Figs. 5D to 5G are diagrams illustrating an example of changes in the liquid image of the blood purification device in the embodiment at different water removal rates. Figs. 6A to 6C are explanatory diagram illustrating an example of a change in the liquid image displayed by the blood purification device in a modification.

The liquid image 730 shown in Figs. 5A to 5C changes faster than the liquid image 730 shown in Figs. 5D to 5G. That is, the water removal rate in Figs. 5A to 5C is faster than that in Figs. 5D to 5G.

The liquid image 730 shown in Figs. 5A to 5G depicts the main display area 71 as a water tank surrounded by the top surface 71a, the bottom surface 71b, a left surface 71c and the right surface 71d, and imitates waves hitting the left surface 71c and the right surface 71d of the main display area 71 and propagating.

On the other hand, Figs. 6A to 6C imitate images as if waves in the center of the water tank are enlarged, not those being reflected at the right surface 71d of the main display area 71.

For the water removal rate, the speed may be expressed, e.g., by changing at least one of an amplitude A of the wave and a wavelength B, as shown in Fig. 3A. When the water removal rate is fast, the amplitude A may be increased, or the wavelength B may be shortened, or both may be performed, as compared to when the rate is slow.

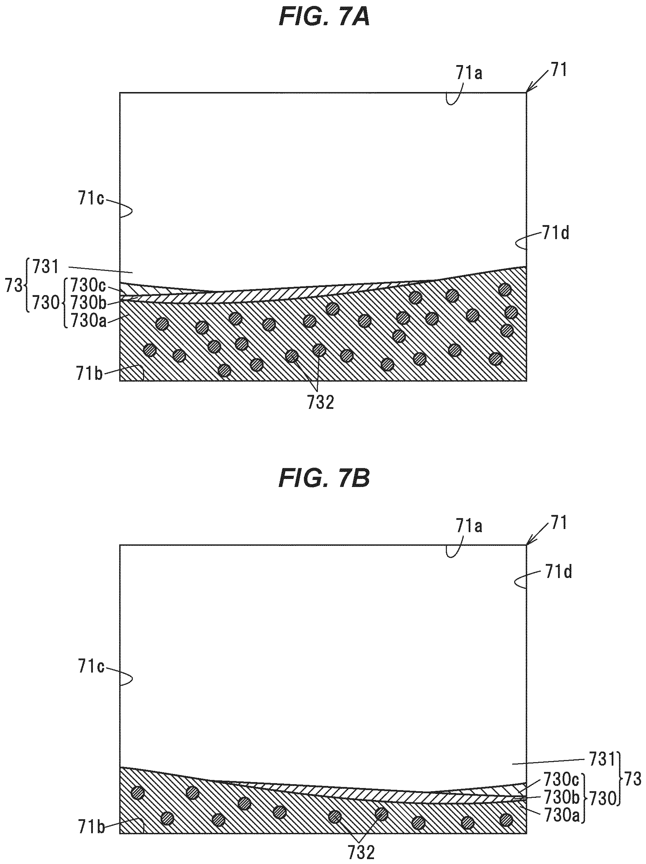

Figs. 7A and 7B are diagrams illustrating an example of when a toxin image is superimposed on the liquid image of the blood purification device in a modification.

Here, as a modification, the control unit 8 may display circular toxin images 732 imitating toxin such as urea, which need to be removed, in the liquid image 730, as shown in Figs. 7A and 7B. The number or density of the toxin images 732 changes according to the processing status of the blood purification treatment, as shown in Figs. 7A and 7B. The toxin images 732 decrease in number and density as the blood purification treatment progresses.

As another modification, the control unit 8 may change the color of the liquid image 730 according to the fluid processing status. For example, the control unit 8 may change the color of the liquid image 730 from yellow to light blue according to the degree of toxin removal. As a further modification, to express the state of blood cells in blood, the control unit 8 may change the color of the liquid image 730 from light blue to yellow according to an amount of blood leakage, which is an amount of blood mixed in the dialysate $L_4$ and is measured by a blood leakage sensor 43a arranged near the dialysate discharge port 53 as shown in Fig. 2B. In this regard, these color changes are just examples and other colors may be used.

As yet another modification, the control unit 8 may be configured to change the color of the background image 73 to inform of an abnormality when occurred.

As still yet another modification, the control unit 8 may be configured to control the height of the liquid surface 73a, i.e., the water level, of the liquid image 730 based on the measured operation amount of the water removal pump 44.

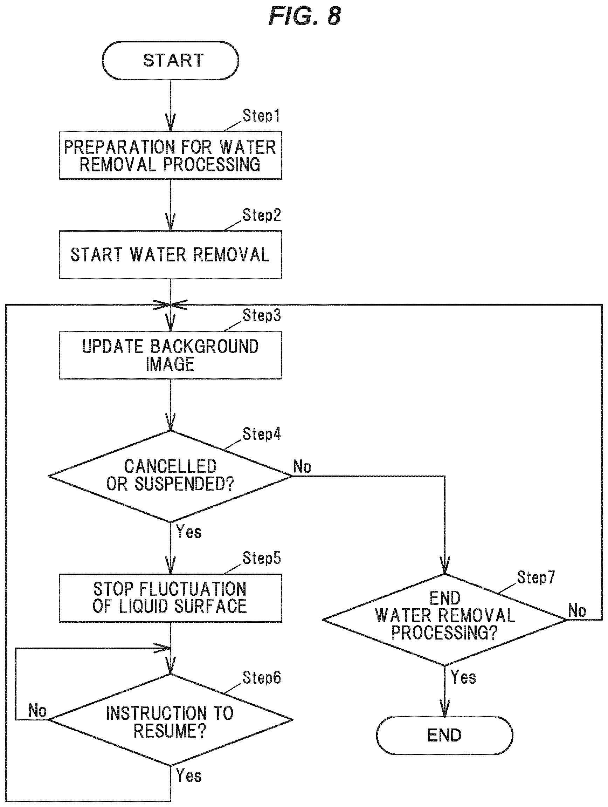

Next, an example of an operation of the blood purification device 1 in the present embodiment related to the water removal processing will be described in reference to the flowchart in Fig. 8.

Operation

The control unit 8 of the blood purification device 1 prepares for the water removal processing (Step 1). The control unit 8 outputs display control information $S_4$ to cause the display/input unit 7 to display the background image 73, based on the first management information $S_1$ obtained from the dialysate dilution unit 3, the second management information $S_2$ obtained from the dialysate supply/discharge unit 4, the third management information $S_3$ obtained from the extracorporeal circulation unit 6, and the processing status of the water removal processing by the water removal unit 4a. The display/input unit 7 displays the background image 73 with a current water removal amount of 0%, based on the display control information $S_4$.

When an operation is performed on the operation image 712c displayed on the display/input unit 7, the control unit 8 starts the water removal processing (Step 2).

The control unit 8 updates the background image 73 and also updates the values displayed in the display items 713a to 713c and the display items 714a to 714c, based on the obtained first management information $S_1$, second management information $S_2$ and third management information $S_3$ and the processing status of the water removal processing by the water removal unit 4a (Step 3). The control unit 8 updates the water level of the liquid image 730 as the update of the background image 73 according to the water removal processing, and also makes the liquid surface 73a fluctuate in waves.

When it is "Yes" in Step 4, i.e., when the water removal processing is cancelled or suspended based on an operation performed on the display/input unit 7 (Step 4: Yes), the control unit 8 stops the fluctuation of the liquid surface 73a of the liquid image 730 (Step 5). In this regard, this cancelation or suspension is not limited to being performed based on an operation by a user, and may be performed at the discretion of the control unit 8.

When it is "Yes" in Step 6, i.e., when an instruction to resume the operation is given based on an operation performed on the display/input unit 7 (Step 6: Yes), the control unit 8 proceeds to Step 3. The control unit 8 may be configured to issue a warning when no instruction to resume is given for a predetermined period of time.

Here, when the dialysis processing is not cancelled or suspended (Step 4: No) and the water removal processing ends (Step 7: Yes), the control unit 8 ends the process by terminating the water removal processing.

Effects of the embodiment

In blood purification treatment, patients are treated for long periods of time while lying in bed, hence, it is preferable that the processing status can be visually checked from a distance of about 2 m which is the length of a bed. Since the blood purification device 1 in the present embodiment shows the processing status of the water removal processing in the background image 73 by changing the water level of the liquid image 730 or fluctuating the liquid surface 73a, it is easier to visually check even from a distance as compared to the case where this configuration is not adopted.

In the blood purification device 1, the target water removal amount, the current water removal amount and the water removal rate of the water removal processing are represented by the area of the main display area 71, the area of the empty space image 731 and the speed of the change in the liquid surface 73a. Therefore, it is easier to visually check the target water removal amount, the current water removal amount and the water removal rate even from a distance as compared to when expressed by numerical values.

Since the blood purification device 1 can display the processing status of the water removal processing using the entire background image 73, the display area is larger and easier to visually check as compared to the case where the processing status is displayed separately from the background image. Therefore, the blood purification device 1 can be configured to have a more compact display area in the display/input unit 7.

Since the blood purification device 1 stops the fluctuation of the liquid surface 73a of the liquid image 730 when the water removal processing is cancelled or suspended, it is easier to visually recognize the cancellation or suspension as compared to the case where the updating of the values of the target water removal amount, the current water removal amount and the water removal rate is stopped or a text image indicating the cancellation or suspension is displayed.

The blood purification device 1 described above is configured such that the power supply unit 2, the dialysate dilution unit 3, the dialysate supply/discharge unit 4, the water removal unit 4a, the extracorporeal circulation unit 6, the display/input unit 7 and the control unit 8 are housed in a single housing. Next, the blood purification device 1 in which at least the dialysate supply/discharge unit 4 and the extracorporeal circulation unit 6 are separate units will be described as an example.

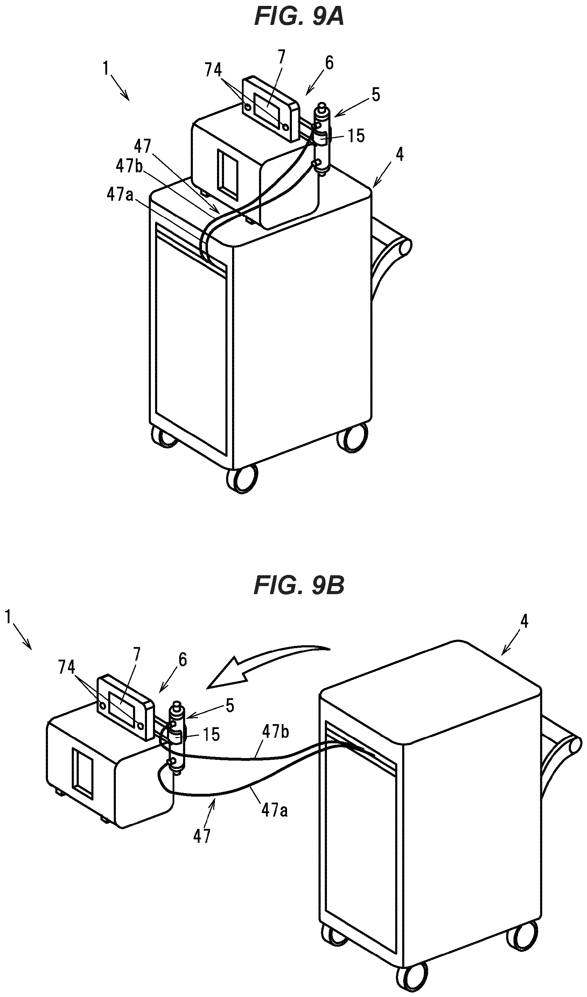

Fig. 9A is an example of a perspective view showing the blood purification device in another embodiment when the extracorporeal circulation unit is attached to the dialysate supply/discharge unit, and Fig. 9B is a perspective view when the extracorporeal circulation unit is detached from the dialysate supply/discharge unit. In the embodiment described below, portions having the same functions and configurations as those in the embodiment described above are denoted by the same reference numerals as those in the embodiment, and the explanation thereof will be omitted.

In this blood purification device 1, the extracorporeal circulation unit 6 is a separate unit from the dialysate supply/discharge unit 4. The control unit 8 can control the dialysate supply/discharge 4 and the extracorporeal circulation unit 6 and is mounted on the extracorporeal circulation unit 6.

The extracorporeal circulation unit 6 has the display/input unit 7 to display a dialysis status, and operating portions 74 to perform an operation related to blood purification treatment. The operating portions 74 may be configured to allow operation inputs different from those available on the display/input unit 7 to be made, or may be configured to allow at least partially common operation inputs to be made. The blood purification device 1 may also be configured such that the display/input unit 7 has only a display function, i.e., is provided as a monitor and operation inputs are made through the operating portions 74. The display/input unit 7 is provided so as to protrude from the top of the extracorporeal circulation unit 6 as shown in Figs. 9A and 9B, but the position or installation layout of the display/input unit 7 is not limited thereto. The display/input unit 7 may be configured to have, e.g., a large display screen 70 that covers the entire surface of the extracorporeal circulation unit 6. In addition, although the operating portions 74 are provided on both sides of the display/input unit 7 in Figs. 9A and 9B, the positions or layout of the operating portions 74 are not limited thereto. By providing the display/input unit 7 and the operating portions 74 on the extracorporeal circulation unit 6 which is relatively lightweight and placed close to the patient, patients can check the treatment status or operate the blood purification device 1 more easily and convenience can thereby be improved.

The display/input unit 7 and the operating portions 74 may be configured as separate components from the extracorporeal circulation unit 6 and can be configured to communicate with the control unit 8 of the extracorporeal circulation unit 6 for display and operation input. In this case, the display/input unit 7 and the operating portions 74 can be composed of, e.g., a dedicated operation terminal, or a portable terminal such as smartphone or tablet. Furthermore, the display/input unit 7 and the operating portions 74 may be mounted on the dialysate supply/discharge unit 4.

The blood purification device 1 shown in Figs. 9A and 9B is configured such that a tube 47 carrying the dialysate $L_4$ and the dialysate effluent $L_6$ directly connects the dialysate supply/discharge unit 4 to the dialyzer 5, without passing through the extracorporeal circulation unit 6. The tube 47 has a supply-side tube 47a to supply the dialysate $L_4$ to the dialyzer 5 and a discharge-side tube 47b to discharge the dialysate effluent $L_6$ from the dialyzer 5. In the blood purification device 1, since the tube 47 carrying the dialysate $L_4$ and the dialysate effluent $L_6$ are not provided on the extracorporeal circulation unit 6, it is only necessary to regularly perform maintenance on the dialysate supply/discharge unit 4 even when the tube 47 carrying the dialysate $L_4$ and the dialysate effluent $L_6$ is a fixed tube, hence, it is possible to achieve both running cost reduction and improvement in maintainability.

Next, an example of a tubing configuration when the blood purification device 1 has the extracorporeal circulation unit 6 and the dialysate supply/discharge unit 4 as separate units will be described.

Fig. 10 is an example block diagram illustrating the blood purification device in the case where the extracorporeal circulation unit and the dialysate supply/discharge unit are separate units. As shown in Fig. 10, the extracorporeal circulation unit 6 and the dialysate supply/discharge unit 4 of the blood purification device 1 are configured as separate units. The dialyzer 5 is removably attached to the extracorporeal circulation unit 6 through a fixing tool 15. The display/input unit 7 is arranged on the extracorporeal circulation unit 6 side and is controlled by a main control unit 8a (described later) as an example, but is not limited thereto. Furthermore, the water removal unit 4a is composed of a liquid supply pump 40a and a liquid discharge pump 49 which are described later.

Configuration of Dialysate supply/discharge unit 4

The dialysate supply/discharge unit 4 has a sub-control unit 8b, a dialysate introduction line 10a to supply the dialysate $L_4$ to the dialyzer 5, a dialysate discharge line 11a to discharge the dialysate effluent $L_6$ from the dialyzer 5, and a bypass flow path 14.

The dialysate introduction line 10a is connected to a dialysate preparation unit 3a and the dialysate introduction port 52 of the dialyzer 5, and supplies the dialysate $L_4$ refined in the dialysate preparation unit 3a to the dialyzer 5. The dialysate discharge line 11a is connected to the dialysate discharge port 53 of the dialyzer 5, and collects and discharges the dialysate $L_4$ from the dialyzer 5. The bypass flow path 14 is connected to the dialysate introduction line 10a and to the dialysate discharge line 11a before and after the dialyzer 5 and connects the dialysate introduction line 10a to the dialysate discharge line 11a while bypassing (avoiding) the dialyzer 5.

That is, the dialysate supply/discharge unit 4 is configured to feed the dialysate $L_4$ in any one of the following liquid feed modes: a main liquid feed mode of feeding the dialysate $L_4$ from the dialysate introduction line 10a to the dialysate discharge line 11a via the dialyzer 5, and a bypass liquid feed mode of feeding the dialysate $L_4$ from the dialysate introduction line 10a to the dialysate discharge line 11a via the bypass flow path 14 without passing through the dialyzer 5.

The dialysate preparation unit 3a prepares the dialysate $L_4$ from pure water supplied thereto and a dialysate agent made of a concentrated solution or powder. The pure water supplied to the dialysate preparation unit 3a may be supplied from a pure water production unit mounted on the dialysate supply/discharge unit 4, or may be supplied from a pure water production device provided outside the dialysate supply/discharge unit 4. In this regard, the dialysate preparation unit 3a can be omitted, and the configuration may be such that, e.g., the dialysate $L_4$ is supplied to the dialysate supply/discharge unit 4 from an external dialysate supply device, etc.

From the upstream side, the liquid supply pump 40a, a flowmeter 41a and a first electromagnetic valve 42a are arranged on the dialysate introduction line 10a. The liquid supply pump 40a is a liquid feed pump that feeds the dialysate $L_4$ in the dialysate introduction line 10a. The flowmeter 41a detects a flow rate in the dialysate introduction line 10a. The first electromagnetic valve 42a is arranged on the downstream side of a connection position connected to the bypass flow path 14 and is one of electromagnetic valves provided to perform switching of the liquid feed mode (flow path) between the main liquid feed mode and the bypass liquid feed mode.

From the upstream side, a second electromagnetic valve 43b, a pressure gauge 44a, a pressure pump 46a, the liquid discharge pump 49 and a flowmeter 48a are arranged on the dialysate discharge line 11a. The pressure pump 46a is an example of a liquid feed pump and an auxiliary pump. The second electromagnetic valve 43b is arranged on the upstream side of a connection position connected to the bypass flow path 14, and is one of the electromagnetic valves provided to perform switching of the liquid feed mode (flow path) between the main liquid feed mode and the bypass liquid feed mode. The pressure gauge 44a detects pressure of the dialysate discharge line 11a. The liquid discharge pump 49 is a liquid feed pump that feeds the dialysate $L_4$ in the dialysate discharge line 11a. The pressure pump 46a is a cascade pump that feeds the dialysate $L_4$ in the dialysate discharge line 11a to assist liquid feeding by the liquid discharge pump 49. The dialysate $L_4$ from the dialyzer 5 is discharged by driving the liquid discharge pump 49 and the pressure pump 46a. The flowmeter 48a detects a flow rate in the dialysate discharge line 11a.

The main control unit 8a adjusts a flow rate of a liquid supplied to the dialyzer 5 and a flow rate of a liquid discharged from the dialyzer 5 by controlling the liquid supply pump 40a and the liquid discharge pump 49 of the water removal unit 4a through the sub-control unit 8b, thereby controlling the amount of water removed from the blood $L_7$ of the patient 9. This blood purification device 1 is capable of performing dialysis treatment without water removal and dialysis treatment with water removal by controlling this water removal amount.

The bypass flow path 14 is arranged such that one end is connected to the dialysate introduction line 10a between the flowmeter 41a and the first electromagnetic valve 42a and the other end is connected between the pressure gauge 44a and the pressure pump 46a. A third electromagnetic valve 45a is arranged on the bypass flow path 14.

The sub-control unit 8b communicates with the main control unit 8a of the extracorporeal circulation unit 6 and controls the liquid supply pump 40a and the liquid discharge pump 49 of the water removal unit 4a, the pressure pump 46a, the first electromagnetic valve 42a, the second electromagnetic valve 43b and the third electromagnetic valve 45a according to commands from the main control unit 8a. The sub-control unit 8b is a microcomputer having a CPU, etc.

Configuration of Extracorporeal circulation unit 6

As shown in Fig. 10, the extracorporeal circulation unit 6 has the main control unit 8a, an arterial blood circuit 12a, and a venous blood circuit 13a. The main control unit 8a is a microcomputer having a CPU, etc. A blood pump 60a to send the blood $L_7$ from the patient 9 to the dialyzer 5 is arranged on the arterial blood circuit 12a. A pressure gauge 61a to detect pressure of the purified blood $L_8$ being returned is arranged on the venous blood circuit 13a.

Description of Main control unit and its control

The main control unit 8a and the control by this main control unit 8a will now be described. The main control unit 8a receives the detection value of the pressure gauge 61a and also controls the drive of the blood pump 60a.

The main control unit 8a also communicates with the sub-control unit 8b, receives detection values of the flowmeter 41a, the flowmeter 48a and the pressure gauge 44a through the sub-control unit 8b and controls drive of the liquid supply pump 40a and the liquid discharge pump 49 of the water removal unit 4a and the pressure pump 46a. Furthermore, the main control unit 8a communicates with the sub-control unit 8b and controls opening and closing of the first electromagnetic valve 42a, the second electromagnetic valve 43b and the third electromagnetic valve 45a through the sub-control unit 8b.

In this regard, the master-subordinate relationship between the main control unit 8a and the sub-control unit 8b is not limited that described above, and the master-subordinate relationship may be reversed or there may be no master-subordinate relationship.

The main control unit 8a drives the blood pump 60a, the liquid supply pump 40a, the pressure pump 46a and the liquid discharge pump 49 in the state in which the liquid feed mode has been switched from the bypass liquid feed mode to the main liquid feed mode by opening and closing the first electromagnetic valve 42a, the second electromagnetic valve 43b and the third electromagnetic valve 45a, and dialysis treatment is thereby performed. That is, during dialysis treatment, the blood pump 60a is driven to circulate the blood $L_7$ through the dialyzer 5 and the liquid supply pump 40a is driven to supply the dialysate $L_4$ to the dialyzer 5, while the pressure pump 46a and the liquid discharge pump 49 are driven to discharge the dialysate $L_4$ from the dialyzer 5.

During this dialysis treatment operation, the flow rate of the liquid supplied to the dialyzer 5 (supply of the dialysate $L_4$) and the flow rate of the liquid discharged from the dialyzer 5 (discharge of the dialysate $L_4$) are adjusted by controlling the liquid supply pump 40a, the pressure pump 46a and the liquid discharge pump 49, thereby controlling the amount of water removed from the blood $L_7$ of the patient 9. In the present embodiment, the pressure pump 46a is driven at a set pump speed (rotational speed), the liquid supply pump 40a is driven by feedback control based on the detection value of the flowmeter 41a so as to achieve the target flow rate, and the liquid discharge pump 49 is driven by feedback control based on the detection value of the flowmeter 48a so as to achieve the target flow rate. The pump speed is an example of a command value of the liquid feed pump.

During preparation before dialysis treatment and during when dialysis treatment is temporarily stopped, the main control unit 8a also controls a dialysate flow operation to make the dialysate $L_4$ flow in the dialysate introduction line 10a and the dialysate discharge line 11a by driving the liquid supply pump 40a, the pressure pump 46a and the liquid discharge pump 49 in the state in which the liquid feed mode has been switched from the main liquid feed mode to the bypass liquid feed mode by controlling opening and closing of the first electromagnetic valve 42a, the second electromagnetic valve 43b and the third electromagnetic valve 45a. During the dialysate flow operation, the pressure pump 46a is driven at a set pump speed, the liquid supply pump 40a is driven by feedback control based on the detection value of the flowmeter 41a so as to achieve the target flow rate, and the liquid discharge pump 49 is driven by feedback control based on the detection value of the flowmeter 48a so as to achieve the target flow rate, in the same manner as during dialysis treatment.

The main control unit 8a changes the liquid surface 73a of the liquid image 730 according to the status of the water removal processing by the water removal unit 4a, i.e., changes in tandem with the speed of the liquid supply pump 40a and the liquid discharge pump 49 of the water removal unit 4a. In more particular, the main control unit 8a changes the extent of the fluctuation of the liquid surface 73a of the liquid image 730 in tandem with the speeds of the liquid supply pump 40a and the liquid discharge pump 49.

Summary of the embodiments

Technical ideas understood from the embodiments will be described below citing the reference signs, etc., used for the embodiments. However, each reference sign, etc., described below is not intended to limit the constituent elements in the claims to the members, etc., specifically described in the embodiments.

(1) A blood purification device 1 comprising: a blood circuit (the arterial blood circuit 12 and the venous blood circuit 13) to extracorporeally circulate blood $L_7$ of a patient 9 through a blood purifier (the dialyzer 5); a tube unit (the dialysate supply/discharge unit 4) through which a liquid comprising a dialysate $L_4$ as a liquid type flows and into which a predetermined component removed from the blood $L_7$ circulating extracorporeally in the blood circuit (the arterial blood circuit 12 and the venous blood circuit 13) is discharged; a display unit (the display/input unit 7) that displays a liquid image 730 imitating a liquid as a background image 73; and a control unit 8 that controls processing of a fluid flowing through at least one of the blood circuit (the arterial blood circuit 12 and the venous blood circuit 13) and the tube unit (the dialysate supply/discharge unit 4), and also controls the display unit (the display/input unit 7) to change a height of a liquid surface 73a of the liquid image 730 in the background image 73 according to a processing status of the fluid in blood purification treatment. This blood purification device makes it easy to visually check the processing status of blood purification treatment by changing the background image.

(2) The blood purification device 1 as defined by (1), wherein the control unit 8 controls the display unit (the display/input unit 7) to change the height of the liquid surface 73a of the liquid image 730 in the background image 73 and also to continuously ripple the liquid surface 73a, according to the processing status of the fluid.

This blood purification device displays the liquid surface so as to continuously ripple according to the fluid processing status. Therefore, it is further easier to visually check the processing status as compared to the case where the liquid surface does not ripple.

(3) The blood purification device 1 as defined by (2), wherein the control unit 8 positions the liquid surface 73a at the highest water level when the processing status of the fluid is 0%, and positions the liquid surface 73a at the lowest water level when the processing status of the fluid is 100%.

This blood purification device can display the fluid processing status using the entire background image. Therefore, the display area is larger and easier to visually check as compared to the case where the processing status is displayed separately from the background image.

(4) The blood purification device 1 as defined by (3), wherein the control unit 8 controls execution of blood purification treatment for exchange of components between blood $L_7$ flowing through the blood circuit (the arterial blood circuit 12 and the venous blood circuit 13) and dialysate $L_4$ flowing through the tube unit (the dialysate supply/discharge unit 4) and, when the blood purification treatment is cancelled or suspended, controls the display unit (the display/input unit 7) to stop fluctuation of the liquid surface 73a of the liquid image 730.

This blood purification device stops the fluctuation of the liquid surface of the liquid image when the blood purification treatment is cancelled or suspended. Therefore, it is easier to visually recognize the cancellation or suspension of the blood purification treatment as compared to the case where the fluctuation is not stopped.

(5) The blood purification device 1 as defined by (4), wherein the control unit 8 changes a color of the liquid image 730 according to the processing status of the fluid.

In this blood purification device, the color of the liquid image changes according to the fluid processing status.

Therefore, it is easier to visually check the fluid processing status as compared to the case where the color does not change.

(6) The blood purification device 1 defined as any one of (1) to (5), further comprising: a water removal unit 4a that, when driven, removes water from blood $L_7$ extracorporeally circulating in the blood circuit (the arterial blood circuit 12 and the venous blood circuit 13) so that the water is discharged into the tube unit (the dialysate supply/discharge unit 4), wherein the processing status of the fluid is a status of water removal processing by the water removal unit 4a, and wherein the control unit 8 changes the liquid image 730 according to the status of the water removal processing by the water removal unit 4a. In this blood purification device, the fluid processing is the water removal processing. Therefore, the target water removal amount, the current water removal amount and the water removal rate are expressed by the changes in the liquid image and are easily visually checked even from a distance.

(7) The blood purification device 1 defined as (6), wherein the control unit 8 changes the liquid surface 73a of the liquid image 730 in tandem with a speed of at least one pump (the water removal pump 44, or the liquid supply pump 40a and the liquid discharge pump 49) of the water removal unit 4a.

In this blood purification device, the liquid surface changes in tandem with the speed of the pump of the water removal unit. Therefore, it is easier to visually check the speed of the water removal processing as compared to the case where this configuration is not adopted.

(8) The blood purification device 1 as defined by (7), wherein the control unit 8 changes the extent of the fluctuation of the liquid surface 73a of the liquid image 730 in tandem with a speed of at least one pump (the water removal pump 44, or the liquid supply pump 40a and the liquid discharge pump 49) of the water removal unit 4a.

In this blood purification device, the liquid surface fluctuates in tandem with the speed of the pump of the water removal unit. Therefore, it is further easier to visually check the speed of the water removal processing as compared to the case where this configuration is not adopted.

(9) The blood purification device 1 as defined by (6), wherein the control unit 8 controls execution of blood purification treatment for exchange of components between blood $L_7$ flowing through the blood circuit (the arterial blood circuit 12 and the venous blood circuit 13) and dialysate $L_4$ flowing through the tube unit (the dialysate supply/discharge unit 4) and causes the display unit (the display/input unit 7) to display a display target image (the display items 713a to 713c and the display items 714a to 714c), which is to be displayed during the blood purification treatment, so as to be superimposed on the liquid image 730.

This blood purification device superimposes the display target image on the background image. Therefore, visibility of the display target image is improved together with visibility of the fluid processing status, as compared to the case where an image related to the fluid processing is displayed separately from the display target image.

Although some embodiments and modifications of the invention have been described, these embodiments and modifications are merely examples and the invention according to claims is not to be limited thereto. These new embodiments and modifications may be implemented in various other forms, and various omissions, substitutions and changes, etc., can be made without departing from the gist of the invention. In addition, not all combinations of the features described in these embodiments and modifications are necessary to solve the problem of the invention. Further, these embodiments and modifications are included within the scope and gist of the invention and also within the invention described in the claims and the range of equivalency.

REFERENCE SIGNS LIST

1: blood purification device, 2: power supply unit, 3: dialysate dilution unit, 4: dialysate supply/discharge unit, 4a: water removal unit, 5: dialyzer, 6: extracorporeal circulation unit, 7: display/input unit, 8: control unit, 32: concentration meter, 33: thermometer, 40: dual pump, 40a: liquid supply pump, 41: pressure gauge, 42: pressure gauge, 43: pressure gauge, 44: water removal pump, 45: flowmeter, 49: liquid discharge pump, 60: blood pump, 61: syringe pump, 62: flowmeter, 63: flowmeter, 70: display screen, 71: main display area, 72: sub-display area, 73: background image, 73a: liquid surface, 720: mode image, 730: liquid image, 730a - 730c: first wave image - third wave image, 731: empty space image, 732: toxin image

**Claims**

1.  A blood purification device, comprising:

    a blood circuit to extracorporeally circulate blood of a patient through a blood purifier;
    a tube unit through which a liquid comprising a dialysate as a liquid type flows and into which a predetermined component removed from the blood circulating extracorporeally in the blood circuit is discharged;

a display unit that displays a liquid image imitating a liquid as a background image; and
a control unit that controls processing of a fluid flowing through at least one of the blood circuit and the tube unit, and also controls the display unit to change a height of a liquid surface of the liquid image in the background image according to a processing status of the fluid.

2. The blood purification device according to claim 1, wherein the control unit controls the display unit to change the height of the liquid surface of the liquid image and also to continuously ripple the liquid surface, according to the processing status of the fluid.

3. The blood purification device according to claim 2, wherein the control unit positions the liquid surface at the highest water level when the processing status of the fluid is 0%, and positions the liquid surface at the lowest water level when the processing status of the fluid is 100%.

4. The blood purification device according to claim 3, wherein the control unit controls execution of blood purification treatment for exchange of components between blood flowing through the blood circuit and dialysate flowing through the tube unit and, when the blood purification treatment is cancelled or suspended, controls the display unit to stop fluctuation of the liquid surface of the liquid image.

5. The blood purification device according to claim 4, wherein the control unit changes a color of the liquid image according to the processing status of the fluid.

6. The blood purification device according to any one of claims 1 to 5, further comprising:

   a water removal unit that, when driven, removes water from blood extracorporeally circulating in the blood circuit so that the water is discharged into the tube unit,
   wherein the processing status of the fluid is a status of water removal processing by the water removal unit, and
   wherein the control unit changes the liquid image according to the status of the water removal processing by the water removal unit.

7. The blood purification device according to claim 6, wherein the control unit changes the liquid surface of the liquid image in tandem with a speed of at least one pump of the water removal unit.

8. The blood purification device according to claim 7, wherein the control unit changes the extent of the fluctuation of the liquid surface of the liquid image in tandem with a speed of at least one pump of the water removal unit.

9. The blood purification device according to claim 6, wherein the control unit controls execution of blood purification treatment for exchange of components between blood flowing through the blood circuit and dialysate flowing through the tube unit and causes the display unit to display a display target image, which is to be displayed during the blood purification treatment, so as to be superimposed on the liquid image.

## FIG. 1A

## FIG. 1B

EP 4 548 943 A1

## FIG. 2A

## FIG. 2B

## FIG. 3A

## FIG. 3B

FIG. 4A

FIG. 4B

FIG. 4C

FIG. 4D

FIG. 5A

FIG. 5B

FIG. 5C

FIG. 5D

*FIG. 5E*

73
730 731    71a 73a                71

71d

71c              71b

*FIG. 5F*

73
730 731    71a 73a                71

71d

71c              71b

*FIG. 5G*

73
730 731    71a 73a              71

71c              71b        71d

*FIG. 6A*

*FIG. 6B*

*FIG. 6C*

## FIG. 7A

## FIG. 7B

## FIG. 8

START

Step1
PREPARATION FOR WATER
REMOVAL PROCESSING

Step2
START WATER REMOVAL

Step3
UPDATE BACKGROUND
IMAGE

Step4
CANCELLED
OR SUSPENDED?　　No

Yes

Step5
STOP FLUCTUATION
OF LIQUID SURFACE

Step6
No　　INSTRUCTION TO
RESUME?

Yes

Step7
END
WATER REMOVAL
PROCESSING?　　No

Yes

END

23

## FIG. 9A

## FIG. 9B

## FIG. 10

## INTERNATIONAL SEARCH REPORT

| | International application No. |
|---|---|
| | **PCT/JP2023/023961** |

### A. CLASSIFICATION OF SUBJECT MATTER

*A61M 1/14*(2006.01)i; *A61M 1/16*(2006.01)i
FI: A61M1/14 110; A61M1/16 111

According to International Patent Classification (IPC) or to both national classification and IPC

### B. FIELDS SEARCHED

Minimum documentation searched (classification system followed by classification symbols)

A61M1/14; A61M1/16

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | US 2018/0193548 A1 (GAMBRO LUNDIA AB) 12 July 2018 (2018-07-12) paragraphs [0037]-[0144], fig. 1-9 | 1-6, 9 |
| A | paragraphs [0037]-[0144], fig. 1-9 | 7-8 |
| A | JP 2012-254250 A (NIKKISO CO LTD) 27 December 2012 (2012-12-27) entire text, all drawings | 1-9 |
| A | US 2016/0038665 A1 (GAMBRO LUNDIA AB) 11 February 2016 (2016-02-11) entire text, all drawings | 1-9 |

☐ Further documents are listed in the continuation of Box C.      ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **18 July 2023** | **01 August 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2023/023961**

| Patent document cited in search report | | | Publication date (day/month/year) | Patent family member(s) | | | Publication date (day/month/year) |
|---|---|---|---|---|---|---|---|
| US | 2018/0193548 | A1 | 12 July 2018 | WO 2017/001556 A1 p. 10, line 19 to p. 60, line 23, fig. 1-9 CN 107847652 A | | | |
| JP | 2012-254250 | A | 27 December 2012 | US 2014/0209519 A1 entire text, all drawings WO 2012/169609 A1 EP 2719405 A1 CN 103596606 A | | | |
| US | 2016/0038665 | A1 | 11 February 2016 | WO 2014/151669 A2 entire text, all drawings CN 104471579 A | | | |

Form PCT/ISA/210 (patent family annex) (January 2015)

27

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004313324 A